# EUROPEAN PATENT APPLICATION

(11) **EP 3 981 321 A1**
(43) Date of publication of application: **13.04.2022**
(21) Application number: 20848466.7
(22) Date of filing: 25.06.2020
(51) Int. Cl.: A61B 5/00

(54) **ELECTROCARDIOGRAM DISPLAY DEVICE, ELECTROCARDIOGRAM DISPLAY METHOD, AND PROGRAM**

(30) Priority: 29.07.2019 JP 2019139026
(71) Applicant: Cardio Intelligence Inc., Tokyo, 106-0044 (JP)
(72) Inventor: TAMURA Yuichi, Tokyo 108-8329 (JP); TANIGUCHI Hirohisa, Tokyo 108-8329 (JP); TANIGUCHI Tadahiro, Kusatsu-shi, Shiga 525-8577 (JP); TAKATA Tomohiro, Yawata-shi, Kyoto 614-8117 (JP)
(74) Representative: Plougmann Vingtoft a/s
(86) International application number: PCT/JP2020/025067
(87) International publication number: WO 2021/019984

(57) **Abstract**

This electrocardiogram display device (3) includes: an input processing unit (341) which inputs divided electrocardiogram data, which is obtained by dividing complete electrocardiogram data obtained by measuring a patient during a prescribed period into the electrocardiogram data of a prescribed time length shorter than the prescribed period, to a machine learning model machine-learnt by using a plurality of teacher electrocardiogram data of the prescribed time length; a result acquisition unit (342) which acquires a determination result that indicates whether a waveform portion thought to indicate heart failure is included in the divided electrocardiogram data output from the machine learning unit (33); and a display control unit (343) which specifies, from the plurality of the divided electrocardiogram data, one or more abnormal electrocardiogram data in which the waveform portion thought to indicate heart failure is included, on the basis of the determination result, and causes a waveform image corresponding to at least some data among the one or more abnormal electrocardiogram data among the specified one or more abnormal electrocardiogram data to be displayed in a doctor's terminal (2).

## Description

### TECHINICAL FIELD

The present disclosure relates to an electrocardiogram display apparatus, a method for displaying an electrocardiogram, and a program.

### BACKGROUND OF THE INVENTION

Conventionally, a Holter monitor that can be worn on the body of a patient to measure a heart rate over a long period is known (see, for example, Patent Document 1).

### PRIOR ART

### PATENT DOCUMENT

[Patent Document 1] Japanese Unexamined Patent Application Publication No. 2007-195693

### SUMMARY OF INVENTION

### PROBLEMS TO BE SOLVED BY THE INVENTION

Conventionally, a laboratory technician has extracted a portion suspected of indicating a disease from an electrocardiogram (ECG) by visually confirming the measured electrocardiogram, and a doctor has provided a diagnosis based on the extracted portion of the electrocardiogram. When the electrocardiogram includes data measured over a long period (e.g., 24 hours), it was difficult to extract a portion where a disease is suspected due to a visual check by the laboratory technician.

Even if the portion where the disease is suspected due to the visual check by the laboratory technician can be extracted, the portion that can be extracted using the conventional method is limited to a portion of waveforms with noticeable abnormality such as a deviation in timing between QRS waveforms or a difference in the shapes of QRS waveforms. In such a situation, there has been a demand for making it easier for a doctor to provide a diagnosis based on a portion of an electrocardiogram suspected of indicating a disease that the laboratory technician is unable to notice.

The present disclosure focuses on this point and its object is to make it easier for a doctor to provide a diagnosis based on a portion in an electrocardiogram suspected of indicating a disease.

### MEANS FOR SOLVING THE PROBLEMS

An electrocardiogram display apparatus according to a first aspect of the present disclosure includes an input processing part that inputs divided electrocardiogram data, which is obtained by dividing whole electrocardiogram data of a patient that has been measured over a predetermined time period into electrocardiogram data having a predetermined time length shorter than the predetermined time period, into a machine learning model realized by machine learning that uses a plurality of pieces of training electrocardiogram data each having the predetermined time length; a result acquisition part that acquires, from the machine learning model, a determination result indicating whether or not the divided electrocardiogram data includes a waveform portion suspected of indicating heart disease; and a display control part that identifies one or more pieces of abnormal electrocardiogram data including the waveform portion suspected of indicating heart disease from a plurality of pieces of the divided electrocardiogram data on the basis of the determination result, and causes a display apparatus to display a waveform image corresponding to at least a part of at least one of the pieces of abnormal electrocardiogram data from among the identified one or more pieces of abnormal electrocardiogram data.

The display control part may cause the display apparatus to display an operation screen for performing a display operation for displaying a waveform image of the abnormal electrocardiogram data other than the abnormal electrocardiogram data whose waveform image is displayed on the display apparatus, when a plurality of pieces of the abnormal electrocardiogram data are identified.

The display control part may cause the display apparatus to display the operation screen including a plurality of pieces of identification information for identifying each of the plurality of pieces of abnormal electrocardiogram data.

The electrocardiogram display apparatus may further include an operation information acquisition part that acquires operation information indicating an operation of selecting one or more pieces of identification information from among the plurality of pieces of identification information, wherein the display control part may cause the display apparatus to simultaneously display a plurality of waveform images corresponding to a plurality of pieces of the abnormal electrocardiogram data on the basis of the operation information acquired by the operation information acquisition part.

The display control part may display information for identifying a waveform portion determined to be suspected of indicating heart disease in the machine learning model together with a waveform image of the abnormal electrocardiogram data in the abnormal electrocardiogram data to be displayed on the display apparatus.

The electrocardiogram display apparatus may further includes a state information acquisition part that acquires (i) state information indicating a state of the patient within the predetermined time period during which the electrocardiogram data is measured and (ii) a time in association with each other, wherein the display control part may cause the display apparatus to display (i) the state indicated by the state information associated with a time at which the abnormal electrocardiogram data was measured along with (ii) a waveform image of the abnormal electrocardiogram data.

In this case, the display control part may display (i) a plurality of pieces of identification information for identifying each of a plurality of pieces of the abnormal electrocardiogram data and (ii) the state in association with each other on the display apparatus.

The display control part may switch between (i) a first mode in which waveform images of a plurality of consecutive pieces of the divided electrocardiogram data including normal electrocardiogram data and abnormal electrocardiogram data which include a waveform portion suspected of indicating heart disease among a plurality of pieces of the divided electrocardiogram data included in the whole electrocardiogram data and (ii) a second mode in which a waveform image of the one or more pieces of the abnormal electrocardiogram data is displayed and a waveform image of the normal electrocardiogram data is not displayed.

An electrocardiogram display apparatus according to a second aspect of the present disclosure includes a display control part that causes a display part to display a waveform image corresponding to at least a part of abnormal electrocardiogram data, which is electrocardiogram data of a fixed time length including a waveform portion suspected of indicating heart disease, among pieces of electrocardiogram data of a patient that have been measured over a predetermined time period, and an operation information acquisition part that acquires operation information for displaying a waveform image corresponding to at least a part of abnormal electrocardiogram data other than the abnormal electrocardiogram data whose waveform image is displayed on the display apparatus, when a plurality of pieces of the abnormal electrocardiogram data are included in the electrocardiogram data.

The display control part may cause the display part to display a plurality of pieces of identification information for identifying each of the plurality of pieces of abnormal electrocardiogram data, and the operation information acquisition part may acquire the operation information indicating an operation of selecting one or more pieces of identification information from among the plurality of pieces of identification information.

A method for displaying an electrocardiogram according to a third aspect of the present disclosure that is executed by a computer includes the steps of: dividing whole electrocardiogram data of a patient that has been measured over a predetermined time period into a plurality of pieces of divided electrocardiogram data having a predetermined time length shorter than the predetermined time period; inputting the plurality of pieces of divided electrocardiogram data into a machine learning model realized by machine learning that uses a plurality of pieces of training electrocardiogram data each having the predetermined time length; acquiring, from the machine learning model, a determination result indicating whether or not the divided electrocardiogram data includes a waveform portion suspected of indicating heart disease; identifying one or more pieces of abnormal electrocardiogram data including the waveform portion suspected of indicating heart disease from a plurality of pieces of the divided electrocardiogram data on the basis of the determination result; and causing a display apparatus to display a waveform image corresponding to at least a part of at least one of the pieces of abnormal electrocardiogram data from among the identified one or more pieces of abnormal electrocardiogram data.

A method for displaying an electrocardiogram according to a fourth aspect of the present disclosure that is executed by a computer includes the steps of: causing a display apparatus to display a waveform image corresponding to at least a part of abnormal electrocardiogram data, which is electrocardiogram data of a fixed time length including a waveform portion suspected of indicating heart disease, among pieces of electrocardiogram data of a patient that have been measured over a predetermined time period, and accepting a display operation for displaying a waveform image corresponding to at least a part of the abnormal electrocardiogram data other than the abnormal electrocardiogram data whose waveform image is displayed on the display apparatus when a plurality of pieces of the abnormal electrocardiogram data are included in the electrocardiogram data.

A program according to a fifth aspect of the present disclosure causes a computer to function as: an input processing part that inputs divided electrocardiogram data, which is obtained by dividing whole electrocardiogram data of a patient that has been measured over a predetermined time period into electrocardiogram data having a predetermined time length shorter than the predetermined time period, into a machine learning model realized by machine learning that uses a plurality of pieces of training electrocardiogram data each having the predetermined time length; a result acquisition part that acquires, from the machine learning model, a determination result indicating whether or not the divided electrocardiogram data includes a waveform portion suspected of indicating heart disease; and a display control part that identifies one or more pieces of abnormal electrocardiogram data including the waveform portion suspected of indicating heart disease from a plurality of pieces of the divided electrocardiogram data on the basis of the determination result, and causes a display apparatus to display a waveform image corresponding to at least a part of at least one of the pieces of abnormal electrocardiogram data from among the identified one or more pieces of abnormal electrocardiogram data.

A program according to a sixth aspect of the present disclosure causes a computer to function as: a display control part that causes a display part to display a waveform image corresponding to at least a part of abnormal electrocardiogram data, which is electrocardiogram data of a fixed time length including a waveform portion suspected of indicating heart disease, among pieces of electrocardiogram data of a patient that have been measured over a predetermined time period, and an operation information acquisition part that accepts a display operation for displaying a waveform image corresponding to at least a part of the abnormal electrocardiogram data other than the abnormal electrocardiogram data whose waveform image is displayed on the display apparatus when a plurality of pieces of the abnormal electrocardiogram data are included in the electrocardiogram data.

### EFFECT OF THE INVENTION

According to the present disclosure, an effect of making it easier for a doctor to provide a diagnosis based on a portion in an electrocardiogram suspected of indicating a disease.

### BRIEF DESCRIPTION OF THE DRAWINGS

FIG. 1 illustrates an outline of an electrocardiogram display system.
FIG. 2 shows a configuration of an electrocardiogram display apparatus.
FIG. 3 is a schematic diagram showing a relationship between the whole electrocardiogram and a divided electrocardiogram.
FIG. 4 shows a display screen as a first example of an electrocardiogram displayed on a doctor's device by the display control part.
FIG. 5 shows a display screen as a second example of the electrocardiogram displayed on the doctor's device by the display control part.
FIG. 6 shows a display screen as a third example of the electrocardiogram displayed on the doctor's device by the display control part.
FIGS. 7A and 7B each show a display screen as a fourth example of the electrocardiogram displayed on the doctor's device by the display control part.
FIG. 8 shows a display screen as a fifth example of the electrocardiogram displayed on the doctor's device by the display control part.
FIG. 9 is a flowchart showing operations performed by a control part.
FIG. 10 shows a variation example of the electrocardiogram display system.
FIG. 11 shows a configuration of an electrocardiogram display apparatus according to the variation example.
FIG. 12 shows a display screen in which a state of a patient is displayed along with an abnormal electrocardiogram.
FIG. 13 shows a configuration of the doctor's device functioning as the electrocardiogram display apparatus.

### DETAILED DESCRIPTION OF THE INVENTION

### [Outline of an electrocardiogram (ECG) display system S]

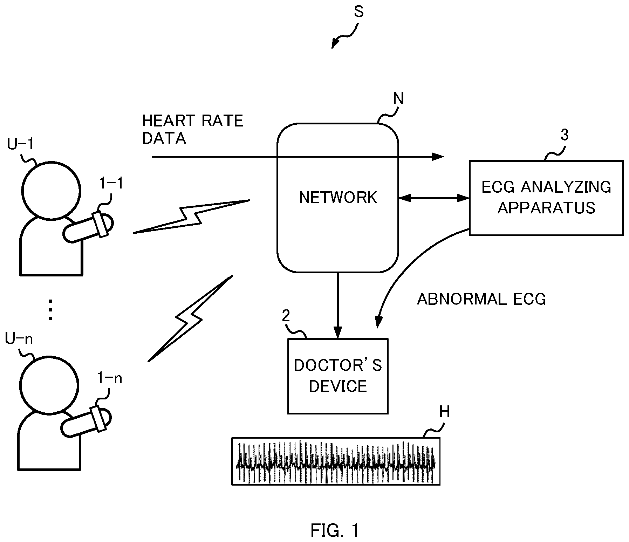

FIG. 1 illustrates an outline of an electrocardiogram display system S. The electrocardiogram display system S is a system for making it easier for a doctor to diagnose a patient by using an electrocardiogram of a patient U who may have heart disease. The electrocardiogram display system S includes an electrocardiograph 1 (1-1 to 1-n, n is a natural number), a doctor's device 2, an electrocardiogram display apparatus 3, and an information terminal 4.

The electrocardiograph 1 is a Holter monitor worn by the patient U (U-1 to U-n), and generates electrocardiogram data which indicates a heartbeat waveform by measuring the patient U's pulse while being worn on his/her wrist, for example. The electrocardiograph 1 transmits the generated electrocardiogram data to the electrocardiogram display apparatus 3 via a network N including a wireless communication line, for example. The electrocardiogram data is associated with time information indicating the time at which the heartbeat is measured. The electrocardiogram data generated by the electrocardiograph 1 may be input to the electrocardiogram display apparatus 3 via a storage medium without passing through the network N, for example.

The doctor's device 2 is a terminal used by a doctor, and includes a display and a computer, for example. The doctor's device 2 displays a waveform image based on a part of the electrocardiogram data received from the electrocardiogram display apparatus 3 within the electrocardiogram data generated by the electrocardiograph 1.

The electrocardiogram display apparatus 3 is an apparatus that generates abnormal electrocardiogram data including a portion suspected of indicating heart disease within the electrocardiogram data received from the electrocardiograph 1 or the doctor's device 2, and is a server, for example. The electrocardiogram display apparatus 3 acquires the electrocardiogram data generated by the electrocardiograph 1 and identifies the portion suspected of indicating disease in the acquired electrocardiogram data using a machine learning model, for example. The machine learning model is a model created by machine learning (for example, deep learning) a large number of pieces of normal electrocardiogram data (i.e., electrocardiogram data in which a disease does not appear) and abnormal electrocardiogram data (i.e., electrocardiogram data in which a disease appears) as training data. An internal configuration of the machine learning model is any desired configuration, and it includes a convolutional neural network (CNN), for example.

By generating abnormal electrocardiogram data corresponding to a portion of the electrocardiogram data including the portion suspected of indicating disease and by transmitting the generated abnormal electrocardiogram data to the doctor's device 2, the electrocardiogram display apparatus 3 displays a waveform image H of the abnormal electrocardiogram data on the doctor's device 2. The electrocardiogram display apparatus 3 may transmit divided electrocardiogram data attached with information indicating whether the divided electrocardiogram data is abnormal or normal to the doctor's device 2, or may transmit only the abnormal electrocardiogram data to the doctor's device 2. When there are a plurality of portions suspected of indicating disease, the electrocardiogram display apparatus 3 transmits a plurality of pieces of abnormal electrocardiogram data to the doctor's device 2 in association with information indicating the time at which electrocardiogram data corresponding to each of the portions suspected of indicating disease was measured. Hereinafter, the configuration and operation of the electrocardiogram display apparatus 3 will be described in detail.

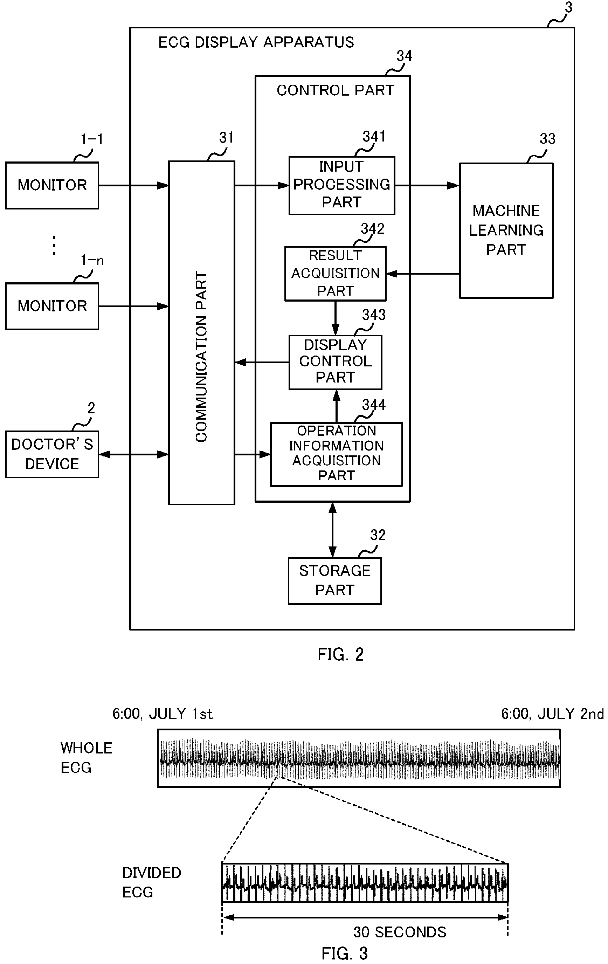

FIG. 2 shows a configuration of the electrocardiogram display apparatus 3. The electrocardiogram display apparatus 3 includes a communication part 31, a storage part 32, a machine learning part 33, and a control part 34. The control part 34 includes an input processing part 341, a result acquisition part 342, a display control part 343, and an operation information acquisition part 344.

The communication part 31 has a communication controller for transmitting and receiving data between the electrocardiograph 1 and the doctor's device 2 via the network N. The communication part 31 notifies the control part 34 of the data received via the network N. The communication part 31 transmits the electrocardiogram data input from the control part 34 to the doctor's device 2 via the network N.

The storage part 32 includes a storage medium such as a read only memory (ROM), a random access memory (RAM), a hard disk, and the like. The storage part 32 stores a program executed by the control part 34. The storage part 32 stores various types of data that are required when the control part 34 executes various calculations.

The machine learning part 33 functions as the above-described machine learning model that can output a result of determining whether or not there is a portion suspected of indicating heart disease in the input electrocardiogram data by learning on the basis of electrocardiogram data for training (hereinafter, training electrocardiogram data), which is to be used as the training data. The machine learning part 33 includes a processor that executes various calculations using the CNN, and a memory that stores coefficients of the CNN, for example. The machine learning part 33 outputs information indicating whether the input electrocardiogram data is normal or abnormal.

The machine learning part 33 may further output abnormal portion information indicating a portion in which a factor with which the input electrocardiogram data is determined to be abnormal electrocardiogram data appears in the input electrocardiogram data. Specifically, when the machine learning part 33 has determined that the input electrocardiogram data is abnormal electrocardiogram data, the machine learning part 33 performs a backpropagation process in which a CNN is traced toward the input end, and then identifies a node where a difference between the input and the output was relatively significant. The machine learning part 33 identifies a node related to a feature that has a significant impact on the determination result using the Grad-CAM method, for example. Such a node is a node related to the feature that has a significant impact on the determination result.

The machine learning part 33 determines that a portion in which the feature corresponding to the identified node appears in the waveform of the abnormal electrocardiogram data is a portion related to the factor with which the input electrocardiogram data is determined to be the abnormal electrocardiogram data. The machine learning part 33 outputs (i) time information corresponding to the portion or (ii) abnormal portion information including information for identifying a portion of the waveform, for example

The control part 34 functions as an input processing part 341, a result acquisition part 342, a display control part 343, and an operation information acquisition part 344 by executing the program stored in the storage part 32.

The input processing part 341 acquires, from the electrocardiograph 1, whole electrocardiogram data of a patient that has been measured over a predetermined time period. The input processing part 341 may acquire the whole electrocardiogram data from the doctor's device 2. The predetermined time period is 24 hours, for example, and it can be any length. The input processing part 341 divides the acquired electrocardiogram data into a plurality of pieces of electrocardiogram data each having a predetermined time length (e.g., 30 seconds) shorter than the predetermined time period. In the present specification, the electrocardiogram data that has been divided is referred to as divided electrocardiogram data.

The electrocardiogram data acquired by the input processing part 341 is any desired electrocardiogram data, and is data in a medical waveform format encoding rules (MFER) format, for example. The input processing part 341 converts the electrocardiogram data in the MFER format into electrocardiogram data in an image format indicating the waveform of the electrocardiogram, and generates the divided electrocardiogram data in the image format.

FIG. 3 is a schematic diagram showing a relationship between the whole electrocardiogram and a divided electrocardiogram. As shown in FIG. 3, when the whole electrocardiogram is an electrocardiogram for 24 hours and the divided electrocardiogram is an electrocardiogram for 30 seconds, the number of divided electrocardiograms is 24 hours × 3600 seconds / 30 seconds = 2880. The input processing part 341 assigns identification numbers (hereinafter, referred to as "divided electrocardiogram IDs") to the divided electrocardiograms in order to identify the divided electrocardiograms, and stores the divided electrocardiogram data in the storage part 32 in association with the divided electrocardiogram IDs. The divided electrocardiogram IDs are numerical values from 1 to 2880, indicating the order of the divided electrocardiograms in the whole electrocardiogram, for example.

The input processing part 341 inputs the divided electrocardiogram data to the machine learning part 33 functioning as a machine learning model. The machine learning model is created by machine learning that uses training electrocardiogram data having the same length as the divided electrocardiogram data as a plurality of pieces of training electrocardiogram data having a predetermined time length, for example. Because the machine learning model is created by machine learning that uses the training electrocardiogram data having the same length as the divided electrocardiogram data, even when the whole electrocardiogram data includes electrocardiogram data measured over a long period, the accuracy of determining the presence or absence of abnormality is improved.

The result acquisition part 342 acquires, from the machine learning model of the machine learning part 33, a determination result indicating whether or not the divided electrocardiogram data includes a waveform portion suspected of indicating heart disease. The result acquisition part 342 notifies the display control part 343 of the determination result.

The result acquisition part 342 may store, in the storage part 32, a divided electrocardiogram ID in association with the determination result indicating whether or not the waveform portion suspected of indicating heart disease is included. For example, the result acquisition part 342 stores divided electrocardiogram data including the waveform portion suspected of indicating heart disease in association with the divided electrocardiogram ID in the storage part 32, and does not store divided electrocardiogram data not including the waveform portion suspected of indicating heart disease in association with the divided electrocardiogram ID in the storage part 32. Since the result acquisition part 342 operates in this manner, the capacity of the storage part 32 is less likely to be insufficient.

The display control part 343 causes the doctor's device 2, functioning as a display apparatus, to display the waveform image of the electrocardiogram data. For example, the display control part 343 transmits the abnormal electrocardiogram data to the doctor's device 2 in order to cause the doctor's device 2 to display a waveform image corresponding to at least a part of the abnormal electrocardiogram data including the waveform portion suspected of indicating heart disease among the plurality of pieces of divided electrocardiogram data. The waveform image corresponding to at least a part of the abnormal electrocardiogram data is (i) the entire waveform image (for example, an image of a waveform for 30 seconds) of the waveform image corresponding to the abnormal electrocardiogram data or (ii) a part of the waveform image (for example, an image of a waveform for 15 seconds) within the waveform image corresponding to the abnormal electrocardiogram data.

In order to transmit the abnormal electrocardiogram data to the doctor's device 2, the display control part 343 identifies one or more pieces of abnormal electrocardiogram data including the waveform portion suspected of indicating heart disease from the plurality of pieces of divided electrocardiogram data on the basis of the determination result acquired from the machine learning model by the result acquisition part 342. The display control part 343 causes the doctor's device 2 to display a waveform image of at least one of the pieces of abnormal electrocardiogram data from among the identified one or more pieces of abnormal electrocardiogram data. That is, the display control part 343 causes the doctor's device 2 to display the waveform image corresponding to at least a part of the divided electrocardiogram data including the waveform portion determined to be suspected of indicating heart disease in the machine learning model.

When the plurality of pieces of abnormal electrocardiogram data are identified, the display control part 343 causes the doctor's device 2 to display an operation screen for performing a display operation for displaying a waveform image corresponding to at least a part of the abnormal electrocardiogram data other than the abnormal electrocardiogram data whose waveform image is displayed on the doctor's device 2. The display control part 343 causes the doctor's device 2 to display an operation screen including the divided electrocardiogram IDs, which are a plurality of pieces of identification information for identifying each of the plurality of pieces of abnormal electrocardiogram data, for example. The screen displayed on the doctor's device 2 by the display control part 343 will be described in detail below.

The operation information acquisition part 344 acquires operation information indicating the contents of operation performed on the doctor's device 2 by a doctor who views the electrocardiogram in the doctor's device 2. The operation information acquisition part 344 acquires, from the doctor's device 2, operation information indicating an operation of selecting one or more divided electrocardiogram IDs from among the plurality of divided electrocardiogram IDs in a state where the display control part 343 causes the doctor's device 2 to display an operation screen for selecting abnormal electrocardiogram data whose waveform image are to be displayed on the doctor's device 2, for example.

### [Display screen of the electrocardiogram]

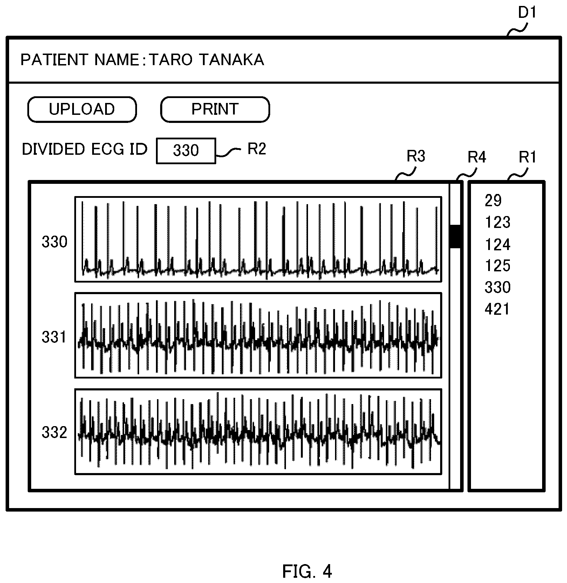

FIG. 4 shows a display screen D1 as a first example of the electrocardiogram displayed on the doctor's device 2 by the display control part 343. An area R1, an area R2, and an area R4 on the display screen D1 are examples of operation screens with which the doctor performs a display operation for displaying other waveform images of abnormal electrocardiogram data.

A divided electrocardiogram ID of abnormal electrocardiogram data is displayed in the area R1 on the display screen D1. In this manner, the display control part 343 causes the doctor's device 2 to display a plurality of divided electrocardiogram IDs for identifying each of the plurality of pieces of abnormal electrocardiogram data. In the example shown in FIG. 4, it is displayed in the area R1 that there are portions suspected of indicating heart disease in the divided electrocardiogram data of which the divided electrocardiogram IDs are 29, 123, 124, 125, 330, and 421. The doctor can select a divided electrocardiogram ID for which the waveform image is to be checked from among the plurality of divided electrocardiogram IDs displayed in the area R1.

The area R2 in FIG. 4 is an area for inputting a divided electrocardiogram ID for which a waveform is to be displayed. The doctor's device 2 displays the waveform image of the divided electrocardiogram data corresponding to the divided electrocardiogram ID input to the area R2. For example, the doctor's device 2 receives all of the divided electrocardiogram data, and displays the waveform image of the divided electrocardiogram data corresponding to the divided electrocardiogram ID input in the area R2 among the received divided electrocardiogram data. The doctor's device 2 may transmit the operation information including the divided electrocardiogram ID input in the area R2 to the electrocardiogram display apparatus 3, acquire the waveform image of the divided electrocardiogram data corresponding to the transmitted divided electrocardiogram ID from the electrocardiogram display apparatus 3, and display the acquired waveform image of the divided electrocardiogram data.

A waveform image of divided electrocardiogram data is displayed in an area R3. The display control part 343 causes the doctor's device 2 to display a waveform image of at least one piece of abnormal electrocardiogram data on the basis of the display operation accepted by an operation accepting part. In the example shown in FIG. 4, the display control part 343 displays a waveform image of divided electrocardiogram data corresponding to the divided electrocardiogram ID input to the area R2 along with waveform images of divided electrocardiogram data acquired in the time periods adjacent to the divided electrocardiogram data. In this manner, the display control part 343 causes the doctor's device 2 to display the waveform image of the divided electrocardiogram data corresponding to the selected divided electrocardiogram ID along with the waveform images of the divided electrocardiogram data in the adjacent time periods. This enables the doctor to provide a diagnosis based on the portion suspected of indicating heart disease while comparing that portion with other portions.

When the abnormal electrocardiogram data continues beyond a default number (3 in the case of FIG. 4) of pieces of data that can be displayed simultaneously on the screen of the doctor's device 2, the display control part 343 may increase the number of pieces of divided electrocardiogram data to be displayed on the screen of the doctor's device 2 beyond the default number so that normal electrocardiogram data can be displayed along with the abnormal electrocardiogram data.

The area R4 is an area for performing an operation of switching the divided electrocardiogram data whose waveform image is displayed in the area R3. The doctor can switch the divided electrocardiogram data whose waveform image is displayed by moving an operation bar shown in black in the area R4 in the vertical direction. For example, when the operation information acquisition part 344 acquires operation information indicating that the operation bar is moved upward, the display control part 343 causes the doctor's device 2 to display a waveform image of the divided electrocardiogram data that was acquired at a time prior to the time when the divided electrocardiogram data whose waveform image is displayed was acquired. In this manner, the doctor can check the desired divided electrocardiogram data using the operation bar.

The "upload" icon in FIG. 4 is used when uploading the whole electrocardiogram data stored in the doctor's device 2 to the electrocardiogram display apparatus 3. When the doctor wants to check the portion suspected of indicating heart disease in the whole electrocardiogram data stored in the doctor's device 2, the whole electrocardiogram data is transmitted from the doctor's device 2 to the electrocardiogram display apparatus 3 and the abnormal electrocardiogram data can be received from the electrocardiogram display apparatus 3 by selecting a file name of the whole electrocardiogram data and selecting the upload icon. A "print" icon in FIG. 4 is used when an operation of printing the displayed electrocardiogram is performed.

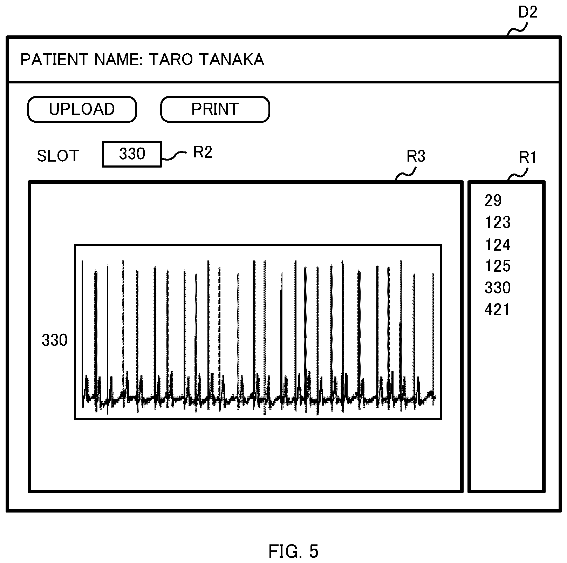

FIG. 5 shows a display screen D2 as a second example of the electrocardiogram displayed on the doctor's device 2 by the display control part 343. In FIG. 5, only the waveform image of the divided electrocardiogram data corresponding to the divided electrocardiogram ID input in the area R2 is displayed. In this manner, the display control part 343 may cause the doctor's device 2 to display only the waveform image of one piece of abnormal electrocardiogram data.

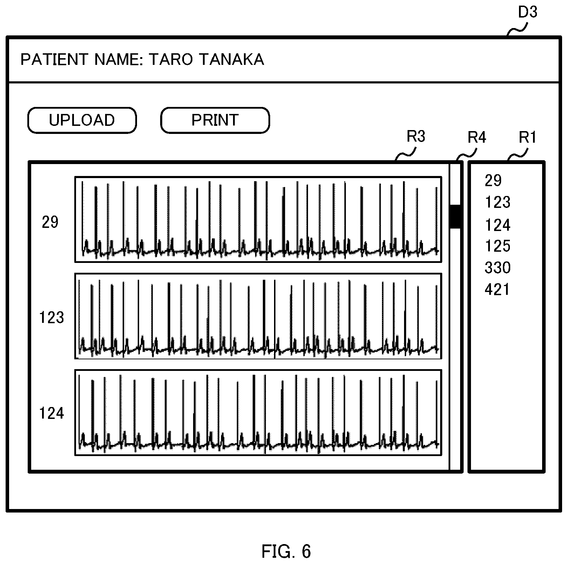

FIG. 6 shows a display screen D3 as a third example of the electrocardiogram displayed on the doctor's device 2 by the display control part 343. In FIG. 6, only the waveform images of the divided electrocardiogram data corresponding to the divided electrocardiogram IDs of the abnormal electrocardiogram data displayed in the area R1 are displayed in the area R3. When the operation bar is moved in the vertical direction, the display control part 343 switches the abnormal electrocardiogram data to be displayed on the doctor's device 2 among the plurality of pieces of abnormal electrocardiogram data.

For example, when the operation information acquisition part 344 acquires operation information indicating that the operation bar is moved upward, the display control part 343 causes the doctor's device 2 to display a waveform image of the abnormal electrocardiogram data that was acquired at a time prior to the time when the abnormal electrocardiogram data whose waveform image is displayed was acquired. As described above, the display control part 343 causes the doctor's device 2 to simultaneously display the waveform images of the plurality of pieces of abnormal electrocardiogram data on the basis of the operation information acquired by the operation information acquisition part 344. This enables the doctor to easily grasp the trend of the abnormality appearing in the electrocardiogram of the patient.

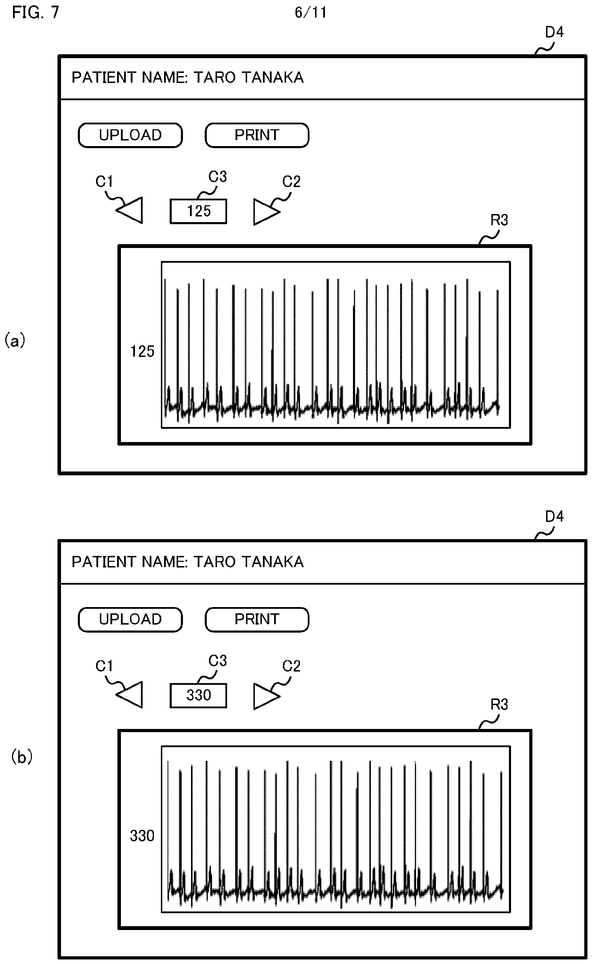

FIGS. 7A and 7B each show a display screen D4 as a fourth example of the electrocardiogram displayed on the doctor's device 2 by the display control part 343.

In FIGS. 7A and 7B, the area R1 in which the divided electrocardiogram IDs of the plurality of pieces of abnormal electrocardiogram data are displayed in a list is not shown, but the icon images C1 and C2 for switching the waveform images to be displayed and an area C3 for displaying the divided electrocardiogram ID for the abnormal electrocardiogram data being on display are shown. The icon image C1 is an image for the doctor to perform an operation for displaying a waveform image of the abnormal electrocardiogram data that was measured at the time before the abnormal electrocardiogram data whose waveform image is on display. The icon image C2 is an image for the doctor to perform an operation for displaying a waveform image of the abnormal electrocardiogram data that was measured at the time after the abnormal electrocardiogram data whose waveform image is on display.

In FIG. 7A, the waveform image of abnormal electrocardiogram data whose divided electrocardiogram ID is 125 is displayed. When the doctor performs an operation of selecting the icon image C2, a waveform image of abnormal electrocardiogram data whose divided electrocardiogram ID is 330, which was measured after abnormal electrocardiogram data whose divided electrocardiogram ID is 125, is displayed, as shown in FIG. 7B.

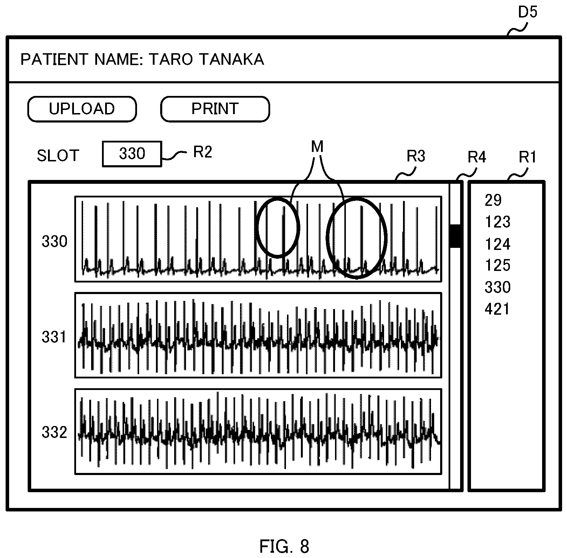

FIG. 8 shows a display screen D5 as a fifth example of the electrocardiogram displayed on the doctor's device 2 by the display control part 343. In FIG. 8, the display control part 343 displays a marker M together with the waveform image of the abnormal electrocardiogram data in the abnormal electrocardiogram data to be displayed on the doctor's device 2. The marker M is information for identifying the waveform portion determined to be suspected of indicating heart disease in the machine learning model. Portions surrounded with the marker M are portions identified by the machine learning part 33 performing the backpropagation process, and are the factors with which the input electrocardiogram data was determined to be abnormal electrocardiogram data. Since the display control part 343 causes the doctor's device 2 to display the information indicating the waveform portion determined to be suspected of indicating heart disease in this manner, even doctors who are not specialized in heart disease can easily grasp the portion that needs to be examined.

The display control part 343 may switch between various types of display modes as shown in FIGS. 4 to 8. For example, the display control part 343 switches between a first mode (e.g., the mode shown in FIGS. 4 and 8) and a second mode (e.g., the mode shown in FIGS. 5, 6, 7A, and 7B).

The first mode is a mode in which the display control part 343 displays waveform images of a plurality of consecutive pieces of divided electrocardiogram data including normal divided electrocardiogram data and abnormal divided electrocardiogram data which include a waveform portion suspected of indicating heart disease, among the plurality of pieces of divided electrocardiogram data included in the whole electrocardiogram data. The second mode is a mode in which the display control part 343 displays a waveform image of one or more pieces of abnormal divided electrocardiogram data and does not display any waveform image of normal divided electrocardiogram data. In this manner, the display control part 343 switches the display mode according to the operation of the doctor who uses the doctor's device 2. This enables the doctor to easily make a proper diagnosis since the abnormal electrocardiogram can be displayed on the doctor's device 2 in a mode suitable for the doctor's purpose in checking the electrocardiogram.

### [Operation flowchart]

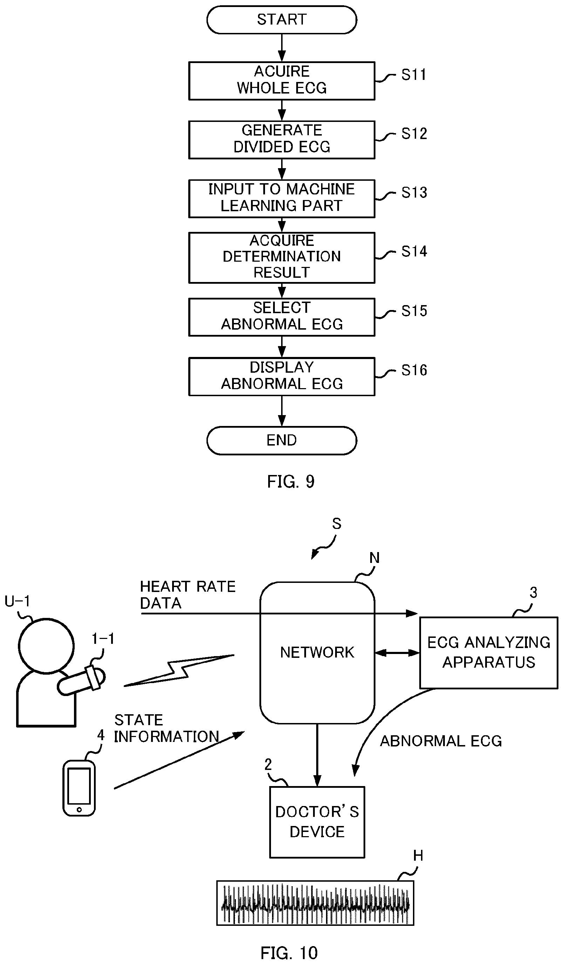

FIG. 9 is a flowchart showing operations performed by the control part 34. The flowchart shown in FIG. 9 starts from the point in time when the input processing part 341 acquires whole electrocardiogram data (S11).

Upon acquiring the whole electrocardiogram data, the input processing part 341 divides the whole electrocardiogram data at predetermined time intervals to generate a plurality of pieces of divided electrocardiogram data (S12). The input processing part 341 inputs the generated plurality of pieces of divided electrocardiogram data to the machine learning part 33 (S13). The result acquisition part 342 acquires, from the machine learning part 33, a result of determining whether or not there is a portion suspected of indicating disease in the divided electrocardiogram data (S14).

Next, the display control part 343 selects abnormal electrocardiogram data from among the plurality of pieces of divided electrocardiogram data on the basis of the determination result acquired by the result acquisition part 342 (S15). In response to a request of the doctor's device 2, the display control part 343 causes the doctor's device 2 to display a waveform image of the selected abnormal electrocardiogram data (S16).

### [Display of a state of the patient U]

FIG. 10 shows a variation example of the electrocardiogram display system S. The electrocardiogram display apparatus 3 shown in FIG. 10 displays, on the doctor's device 2, (i) the patient U's state identified on the basis of information received from the information terminal 4, which the patient U uses, along with (ii) the waveform image of the abnormal electrocardiogram data. The information terminal 4 is a terminal, such as a smartphone, carried and used by the patient U and has various types of sensors for detecting a state of the patient U.

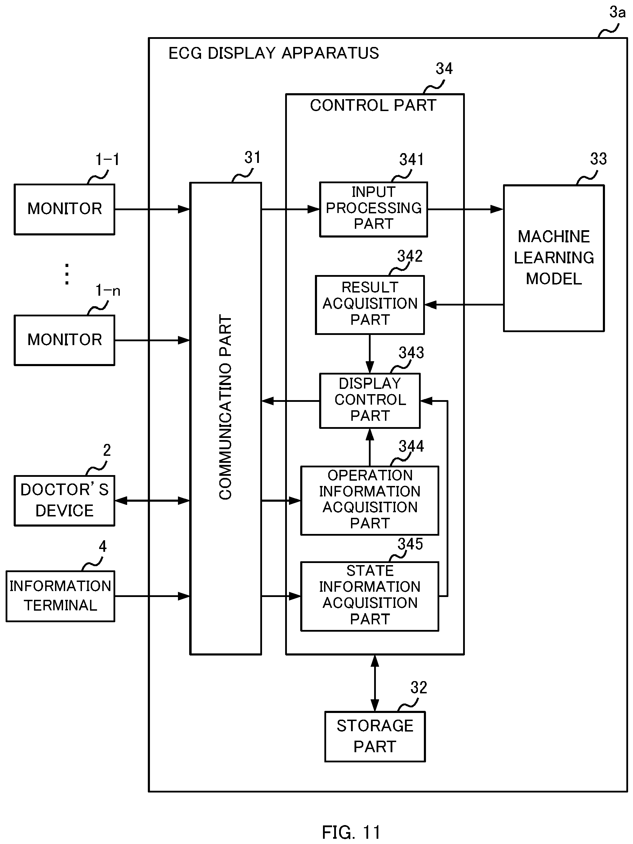

FIG. 11 shows a configuration of an electrocardiogram display apparatus 3a according to the variation example. The electrocardiogram display apparatus 3a shown in FIG. 11 is different from the electrocardiogram display apparatus 3 shown in FIG. 2 in a point that the electrocardiogram display apparatus 3a further includes a state information acquisition part 345, and is the same with respect to the other points.

The state information acquisition part 345 acquires (i) state information indicating the state of the patient U within a predetermined time period during which the electrocardiogram data is measured and (ii) the time in association with each other. The state information acquisition part 345 notifies the display control part 343 of the acquired state information and information indicating the time. The state information acquisition part 345 acquires, from the information terminal 4, information related to the state of the patient U, such as his/her movement, a place where he/she is (latitude, longitude, and altitude), and the temperature or the like of the place where he/she is. Information indicating the movement of the patient U is acceleration detected by an acceleration sensor included in the information terminal 4, for example. Information indicating the place where the patient U is, for example, is information indicating the latitude and longitude specified by a GPS receiver of the information terminal 4 and the altitude detected by an altitude sensor of the information terminal 4.

The display control part 343 identifies the state information corresponding to the time at which the abnormal electrocardiogram data was measured, from among a plurality of pieces of state information associated with a plurality of the times notified from the state information acquisition part 345. The display control part 343 causes the doctor's device 2 to display (i) the state indicated by the state information associated with the time at which the abnormal electrocardiogram data was measured along with (ii) the waveform image of the abnormal electrocardiogram data. The display control part 343 displays (i) the plurality of abnormal electrocardiogram IDs for identifying each of the plurality of pieces of abnormal electrocardiogram data and (ii) the state of the patient U in association with each other on the doctor's device 2.

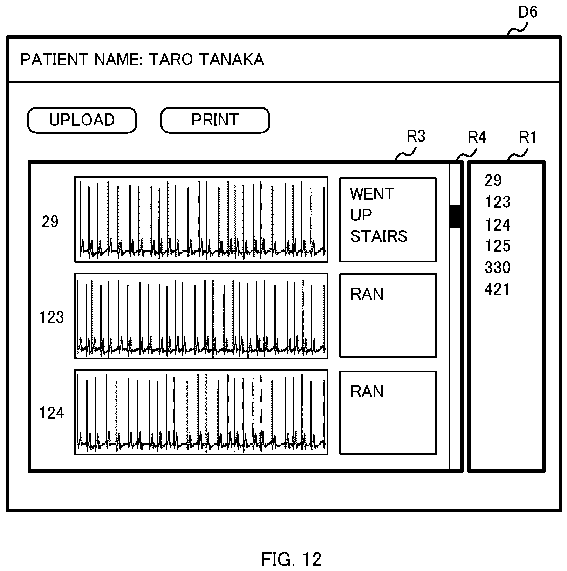

FIG. 12 shows a display screen D6 in which the states of the patient U are displayed along with the abnormal electrocardiograms. In FIG. 12, a state of "went up the stairs" is displayed in association with a waveform image of abnormal electrocardiogram data whose divided electrocardiogram ID is 29, and states of "ran" are displayed in association with waveform images of pieces of abnormal electrocardiogram data whose divided electrocardiogram IDs are 123 and 124. As described above, since the display control part 343 causes the doctor's device 2 to display (i) the waveform images of the abnormal electrocardiograms and (ii) the states of the patient U in association with each other, the doctor can grasp the state of the patient U when the abnormality has occurred, and therefore the doctor can easily make a proper diagnosis.

When the time information associated with the electrocardiogram data and the time information associated with the state information are synchronized with each other, the machine learning part 33 may learn data obtained by combining the electrocardiogram data and the state information as the training data. In this case, the input processing part 341 inputs the divided electrocardiogram data and the state information indicating the state of the patient U at the time when the divided electrocardiogram data was measured to the machine learning part 33. The machine learning part 33 determines whether or not the divided electrocardiogram data input together with the state information includes a portion suspected of indicating heart disease, and outputs a determination result. By having the machine learning part 33 use the state information together with the divided electrocardiogram data in this way, the determination accuracy is further improved.

The input processing part 341 may input only the electrocardiogram data to the machine learning part 33 and notify the display control part 343 about the state information. In this case, even the divided electrocardiogram data determined to be abnormal electrocardiogram data by the machine learning part 33 may be treated as normal electrocardiogram data when it is determined that the divided electrocardiogram data is not abnormal on the basis of the state information.

### [Doctor's device 2 functioning as the electrocardiogram display apparatus]

In the above description, a case where the electrocardiogram display apparatus 3 displays the waveform image of the abnormal electrocardiogram data on the doctor's device 2 on the basis of the result of identifying the portion suspected of indicating heart disease in the electrocardiogram was illustrated as an example. On the other hand, the doctor's device 2 used by the doctor may function as an electrocardiogram display apparatus that identifies a portion suspected of indicating heart disease in the electrocardiogram and displays the electrocardiogram including this portion.

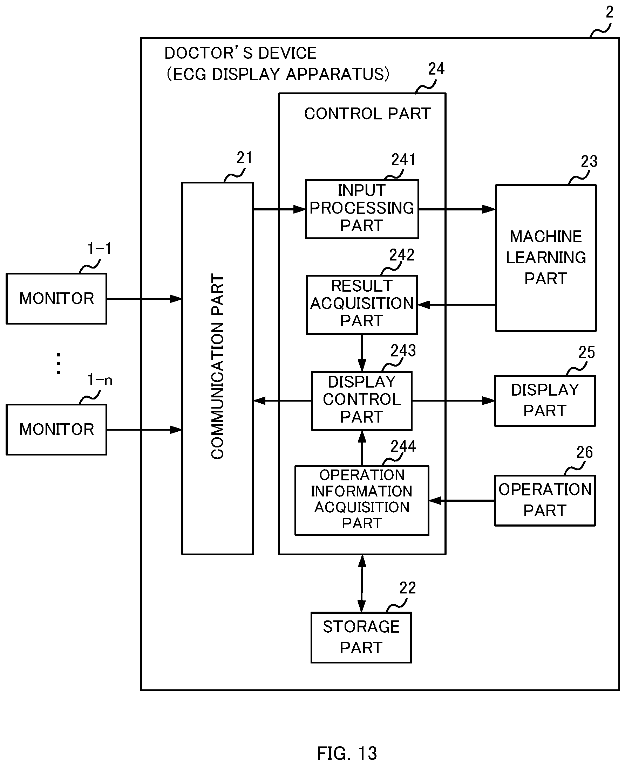

FIG. 13 shows a configuration of the doctor's device 2 functioning as the electrocardiogram display apparatus. The doctor's device 2 shown in FIG. 13 includes a communication part 21, a storage part 22, a machine learning part 23, a control part 24, a display part 25, and an operation part 26. The control part 24 includes an input processing part 241, a result acquisition part 242, a display control part 243, and an operation information acquisition part 244. The communication part 21, the storage part 22, the machine learning part 23, and the control part 24 have the same functions as the communication part 31, the storage part 32, the machine learning part 33, and the control part 34 in the electrocardiogram display apparatus 3 shown in FIG. 2, respectively.

The input processing part 241, the result acquisition part 242, the display control part 243, and the operation information acquisition part 244 have the same functions as the input processing part 341, the result acquisition part 342, the display control part 343, and the operation information acquisition part 344, respectively. In the doctor's device 2, the control part 24 functions as the input processing part 241, the result acquisition part 242, the display control part 243, and the operation information acquisition part 244 as well, by the control part 24 executing a program stored in the storage part 22.

The display control part 243 causes the display part 25 to display a waveform image of abnormal electrocardiogram data, which is electrocardiogram data of a fixed time length including a waveform portion suspected of indicating heart disease, among the pieces of electrocardiogram data of the patient that have been measured over the predetermined time period. The display control part 243 causes the display part 25 to display the divided electrocardiogram IDs, which are the plurality of pieces of identification information for identifying each of the plurality of pieces of abnormal electrocardiogram data.

When the plurality of pieces of abnormal electrocardiogram data are included in the electrocardiogram data, the operation information acquisition part 244 acquires operation information for displaying a waveform image of abnormal electrocardiogram data other than the abnormal electrocardiogram data whose waveform image is displayed on the display part 25. The operation information acquisition part 244 acquires operation information indicating an operation of selecting one or more pieces of identification information from the plurality of pieces of identification information, and notifies the display control part 243 of the acquired operation information. The display control part 243 selects abnormal electrocardiogram data whose waveform image is to be displayed on the display part 25 on the basis of the notified operation information. Since the doctor's device 2 is configured in this manner, the doctor's device 2 can display the abnormal electrocardiogram identified by using the machine learning model even when the doctor's device 2 is not connected to the server via a network.

### [Effect of the electrocardiogram display system S]

As described above, in the electrocardiogram display system S, the display control part 343 causes the doctor's device 2 to display the waveform image of the abnormal electrocardiogram data identified on the basis of the determination result, indicating whether or not the waveform portion suspected of indicating heart disease is included, which is acquired from the machine learning part 33 to which the divided electrocardiogram data is input. Since the electrocardiogram display system S is configured in this manner, even if the doctor using the doctor's device 2 is not a specialist in heart disease, the probability that he/she can properly determine the presence or absence of a disease is increased.

In addition, the display control part 343 causes the doctor's device 2 to display an operation screen for performing a display operation for displaying a waveform image of abnormal electrocardiogram data other than the abnormal electrocardiogram data whose waveform image is displayed on the doctor's device 2. When a plurality of pieces of abnormal electrocardiogram data are included in the electrocardiogram data, the operation information acquisition part 344 acquires operation information for displaying the waveform image of the abnormal electrocardiogram data other than the abnormal electrocardiogram data whose waveform image is displayed on the doctor's device 2.
Since the display control part 343 and the operation information acquisition part 344 operate in this manner, the doctor can easily check a portion suspected of having an abnormality from within the electrocardiogram data acquired over a long period.

The present disclosure is explained based on the exemplary embodiments. The technical scope of the present disclosure is not limited to the scope explained in the above embodiments and it is possible to make various changes and modifications within the scope of the disclosure. For example, all or part of the apparatus can be configured with any unit which is functionally or physically dispersed or integrated. Further, new exemplary embodiments generated by arbitrary combinations of them are included in the exemplary embodiments. Further, effects of the new exemplary embodiments brought by the combinations also have the effects of the original exemplary embodiments.

For example, in the above description, a case where the display control part 343 causes the doctor's device 2 to display the waveform image corresponding to the abnormal electrocardiogram data on the basis of the result of determining by the machine learning part 33 whether or not the electrocardiogram data includes the portion suspected of indicating heart disease has been described. However, the display control part 343 may display the waveform image corresponding to the abnormal electrocardiogram data on the doctor's device 2 without using the machine learning part 33. The display control part 343 may display the waveform image corresponding to the abnormal electrocardiogram data on the doctor's device 2 on the basis of a result of determining whether or not there is an abnormality in the divided electrocardiogram data using an image analysis method that does not use the machine learning model, for example.

### [Description of the reference numerals]

- 1: Electrocardiograph
- 2: Doctor's device
- 3: Electrocardiogram display apparatus
- 4: Information terminal
- 31: Communication part
- 32: Storage part
- 33: Machine learning part
- 34: Control part
- 35: Display part
- 36: Operation part
- 341: Input processing part
- 342: Result acquisition part
- 343: Display control part
- 344: Operation information acquisition part
- 345: State information acquisition part

## Claims

1. An electrocardiogram display apparatus comprising:
an input processing part that inputs divided electrocardiogram data, which is obtained by dividing whole electrocardiogram data of a patient that has been measured over a predetermined time period into electrocardiogram data having a predetermined time length shorter than the predetermined time period, into a machine learning model realized by machine learning that uses a plurality of pieces of training electrocardiogram data each having the predetermined time length;
a result acquisition part that acquires, from the machine learning model, a determination result indicating whether or not the divided electrocardiogram data includes a waveform portion suspected of indicating heart disease; and
a display control part that identifies one or more pieces of abnormal electrocardiogram data including the waveform portion suspected of indicating heart disease from a plurality of pieces of the divided electrocardiogram data on the basis of the determination result, and causes a display apparatus to display a waveform image corresponding to at least a part of at least one of the pieces of abnormal electrocardiogram data from among the identified one or more pieces of abnormal electrocardiogram data.

2. The electrocardiogram display apparatus according to claim 1, wherein the display control part causes the display apparatus to display an operation screen for performing a display operation for displaying a waveform image of the abnormal electrocardiogram data other than the abnormal electrocardiogram data whose waveform image is displayed on the display apparatus, when a plurality of pieces of the abnormal electrocardiogram data are identified.

3. The electrocardiogram display apparatus according to claim 2, wherein the display control part causes the display apparatus to display the operation screen including a plurality of pieces of identification information for identifying each of the plurality of pieces of abnormal electrocardiogram data.

4. The electrocardiogram display apparatus according to claim 2 or 3, further comprising
an operation information acquisition part that acquires operation information indicating an operation of selecting one or more pieces of identification information from among the plurality of pieces of identification information, wherein
the display control part causes the display apparatus to simultaneously display a plurality of waveform images corresponding to a plurality of pieces of the abnormal electrocardiogram data on the basis of the operation information acquired by the operation information acquisition part.

5. The electrocardiogram display apparatus according to any one of claims 1 to 4, wherein the display control part displays information for identifying a waveform portion determined to be suspected of indicating heart disease in the machine learning model together with a waveform image of the abnormal electrocardiogram data in the abnormal electrocardiogram data to be displayed on the display apparatus.

6. The electrocardiogram display apparatus according to any one of claims 1 to 5, further comprising
a state information acquisition part that acquires (i) state information indicating a state of the patient within the predetermined time period during which the electrocardiogram data is measured and (ii) a time in association with each other, wherein
the display control part causes the display apparatus to display (i) the state indicated by the state information associated with a time at which the abnormal electrocardiogram data was measured along with (ii) a waveform image of the abnormal electrocardiogram data.

7. The electrocardiogram display apparatus according to claim 6, wherein the display control part displays (i) a plurality of pieces of identification information for identifying each of a plurality of pieces of the abnormal electrocardiogram data and (ii) the state in association with each other on the display apparatus.

8. The electrocardiogram display apparatus according to any one of claims 1 to 7, wherein the display control part switches between (i) a first mode in which waveform images of a plurality of consecutive pieces of the divided electrocardiogram data including normal electrocardiogram data and abnormal electrocardiogram data which include a waveform portion suspected of indicating heart disease among a plurality of pieces of the divided electrocardiogram data included in the whole electrocardiogram data and (ii) a second mode in which a waveform image of the one or more pieces of the abnormal electrocardiogram data is displayed and a waveform image of the normal electrocardiogram data is not displayed.

9. An electrocardiogram display apparatus comprising:
a display control part that causes a display part to display a waveform image corresponding to at least a part of abnormal electrocardiogram data, which is electrocardiogram data of a fixed time length including a waveform portion suspected of indicating heart disease, among pieces of electrocardiogram data of a patient that have been measured over a predetermined time period; and
an operation information acquisition part that acquires operation information for displaying a waveform image corresponding to at least a part of abnormal electrocardiogram data other than the abnormal electrocardiogram data whose waveform image is displayed on the display apparatus, when a plurality of pieces of the abnormal electrocardiogram data are included in the electrocardiogram data.

10. The electrocardiogram display apparatus according to claim 9, wherein
the display control part causes the display part to display a plurality of pieces of identification information for identifying each of the plurality of pieces of abnormal electrocardiogram data, and
the operation information acquisition part acquires the operation information indicating an operation of selecting one or more pieces of identification information from among the plurality of pieces of identification information.

11. A method for displaying an electrocardiogram that is executed by a computer, comprising the steps of:
dividing whole electrocardiogram data of a patient that has been measured over a predetermined time period into a plurality of pieces of divided electrocardiogram data having a predetermined time length shorter than the predetermined time period;
inputting the plurality of pieces of divided electrocardiogram data into a machine learning model realized by machine learning that uses a plurality of pieces of training electrocardiogram data each having the predetermined time length;
acquiring, from the machine learning model, a determination result indicating whether or not the divided electrocardiogram data includes a waveform portion suspected of indicating heart disease;
identifying one or more pieces of abnormal electrocardiogram data including the waveform portion suspected of indicating heart disease from a plurality of pieces of the divided electrocardiogram data on the basis of the determination result; and
causing a display apparatus to display a waveform image corresponding to at least a part of at least one of the pieces of abnormal electrocardiogram data from among the identified one or more pieces of abnormal electrocardiogram data.

12. A method for displaying an electrocardiogram that is executed by a computer, comprising the steps of:
causing a display apparatus to display a waveform image corresponding to at least a part of abnormal electrocardiogram data, which is electrocardiogram data of a fixed time length including a waveform portion suspected of indicating heart disease, among pieces of electrocardiogram data of a patient that have been measured over a predetermined time period, and
accepting a display operation for displaying a waveform image corresponding to at least a part of the abnormal electrocardiogram data other than the abnormal electrocardiogram data whose waveform image is displayed on the display apparatus when a plurality of pieces of the abnormal electrocardiogram data are included in the electrocardiogram data.

13. A program that causes a computer to function as:
an input processing part that inputs divided electrocardiogram data, which is obtained by dividing whole electrocardiogram data of a patient that has been measured over a predetermined time period into electrocardiogram data having a predetermined time length shorter than the predetermined time period, into a machine learning model realized by machine learning that uses a plurality of pieces of training electrocardiogram data each having the predetermined time length;
a result acquisition part that acquires, from the machine learning model, a determination result indicating whether or not the divided electrocardiogram data includes a waveform portion suspected of indicating heart disease; and
a display control part that identifies one or more pieces of abnormal electrocardiogram data including the waveform portion suspected of indicating heart disease from a plurality of pieces of the divided electrocardiogram data on the basis of the determination result, and causes a display apparatus to display a waveform image corresponding to at least a part of at least one of the pieces of abnormal electrocardiogram data from among the identified one or more pieces of abnormal electrocardiogram data.

14. A program that causes a computer to function as:
a display control part that causes a display part to display a waveform image corresponding to at least a part of abnormal electrocardiogram data, which is electrocardiogram data of a fixed time length including a waveform portion suspected of indicating heart disease, among pieces of electrocardiogram data of a patient that have been measured over a predetermined time period, and
an operation information acquisition part that accepts a display operation for displaying a waveform image corresponding to at least a part of the abnormal electrocardiogram data other than the abnormal electrocardiogram data whose waveform image is displayed on the display apparatus when a plurality of pieces of the abnormal electrocardiogram data are included in the electrocardiogram data.
